# EUROPEAN PATENT SPECIFICATION

(11) **EP 1 408 999 B1**
(45) Date of publication and mention of the grant of the patent: **12.03.2008**
(21) Application number: 00950109.9
(22) Date of filing: 21.07.2000
(51) Int. Cl.: A61K 35/56, A61K 36/15, A61P 29/00

(54) **COMPOSITIONS ADDRESSING INFLAMMATION AND/OR DEGENERATIVE DISORDERS**
MITTEL ZUR BEKÄMPFUNG VON ENTZÜNDUNGEN UND/ODER DEGENERATIVEN ERKRANKUNGEN
COMPOSITIONS DESTINEES AU TRAITEMENT D'INFLAMMATIONS ET/OU D'AFFECTIONS DEGENERATIVES

(30) Priority: 21.07.1999 NZ 33685699; 27.10.1999 NZ 50063099; 21.07.2000 NZ 50587500
(43) Date of publication of application: 21.04.2004
(73) Proprietor: Bomac Research Limited, Auckland 1701 (NZ)
(72) Inventor: HASHMI, Syed, Ziauddin, Auckland 1701 (NZ); LEECH, Wayne, Frederick, Manukau City, Auckland 1701 (NZ); MCLAREN, Donald, George, Auckland 1750 (NZ); MCSPORRAN, Keith, David, Auckland 1003 (NZ)
(74) Representative: Murphy, Colm Damien
(86) International application number: PCT/NZ2000/000135
(87) International publication number: WO 2001/005411

(56) References cited:
- DATABASE WPI Section Ch, Week 199745 Derwent Publications Ltd., London, GB; Class B04, AN 1997-487854 XP002154177 & NZ 270 754 A (MCFARLANE LAB NZ LTD), 22 August 1997 (1997-08-22)

## Description

### TECHNICAL FIELD

The present invention is directed to compositions for addressing degenerative disorders and inflammation. Preferred embodiments of the invention comprises a sustained slow-acting composition which, when continually administered, exhibit anti-inflammatory effects though various embodiments may also exhibit analgesic effects, gastro-protective effects, a reduction in host-cell damage associated with inflammation, and may reduce cancerous tumours through antiangiogenesis. Differing embodiments may exhibit a number, or all, of these effects to varying degrees depending upon the degree and balance of synergism resulting from the selected components and ratios.

### BACKGROUND ART

The present invention was developed with the needs and problems associated with domestic animals in mind. In particular, domestic pets receive significantly more attention from humans than domesticated commercial species (e.g. livestock). The care and attention lavished on domestic pets also means that they tend to live to a significantly greater age than most commercially bred species and are thus more likely to exhibit the problems associated with old age. Such problems include cancer, and debilitating degenerative diseases.

In addition, animals are also susceptible to inflammation associated with various causes such as tissue damage or injury and, as for their human counterparts, some animals may also experience gastro-intestinal irritation from commonly used anti-inflammatories. As many domesticated pets are regarded by owners as family members, owners are often keen to address the various maladies that their pets exhibit

In most cases the solution is a curative remedial action after the problem has presented itself. While this may be effective for temporary afflictions such as acute infectious inflammation, longer term afflictions such as cancer and debilitating degenerative ailments have associated degenerative or other effects which are not usually fully reversible and quite often any remedial action is merely to attempt to control the further spread of the affliction, or to ameliorate its effects on the animal. In some instances a partial improvement may be obtained, though there are problems associated with addressing an affliction after it has firmly_established itself. As for humans, early diagnosis is often associated with a better prognosis for recovery or control.

Accordingly, a number of afflictions such as cancer or debilitating degenerative ailments (e.g. arthritis) may be more effectively controlled if preventative measures are taken. For instance there is evidence indicating that cartilage protecting agents may help protect against the occurrence of degenerative joint diseases and associated complaints. While there are varying forms of joint diseases, in general the complaint is accompanied by degeneration of cartiligenous material at the joints. The sooner action is taken against such degeneration, then the less the effects of the complaint will be. Thus, while an animal may still remain susceptible to joint related afflictions, preventative measures may protect against development of the complaint to any appreciable degree.

Similarly, inflammation at the joint is a factor in some degenerative joint diseases and thus some protection may also be provided by preventing inflammation in affected areas.

There are also a number of different types of cancers, though in particular the present invention is more focussed on those accompanied by tumourous growths. In many instances these tumours may be relatively benign though any tumourous growth is potentially serious. Again there is a link between early prognosis and recovery or effective control of the cancer and thus any preventative measure which can hinder the early growth or development of tumours will be of use.

For most animals, there is a limited number of products available which can be safely administered to afford a preventative or curative action towards these types of afflictions. Most animal remedies are based on pure chemicals for addressing a particular diagnosed chemical imbalance. Many of these contain side effects, and even for those that don't, it is generally not a recommended practice for their regular continued administration.

For domestic pets, there have been on-going improvements in food formulations, though again the primary emphasis has been on presenting a tailored balance of nutrients for different animals. A number of more recent formulations have addressed the elimination of problem components, or have altered the foodstuff characteristics to counter known problems in pets - for example, altering the pH or certain pelletised cat foods to avoid urinary tract problems in adult cats. Most focus on various vitamins and minerals and may also increase or reduce specific amino acids present in the foodstuff. Some products have become quite specialised and one American product is specifically formulated for dogs undergoing chemotherapy, and includes high levels of n-3 fatty acids, which inhibit tumour growth.

However, there is a general need for a composition which can be administered on a regular basis to both healthy and afflicted animals and which can address one or more of a number of known, common, problems such as indicated above. Accordingly it is one aspect of the present invention to provide a composition, in a dosage form, or an alternate form, which can be administered regularly and in with relative safety to most domesticated pets, and particular mammalian species. At the very least, it is an object of the present invention to provide the public with a useful alternative to what is currently available.

Further aspects and advantages of the present invention will become apparent from the ensuing description which is given by way of example only.

### DISCLOSURE OF INVENTION

According to a first aspect of the present invention there is provided an anti-inflammatory composition for administration to animals comprising a combination of:
a) a green-lipped mussel extract (GLME) and/or a pharmacologically active green lipped mussel product;
b) a shark cartilage and/or a pharmacologically active shark cartilage; and
c) a bark product or bark extract
wherein the bark product or extract enhances the effect of the anti-inflammatory agents.

Typically the composition includes a range of vitamins and/or trace minerals.

Preferably the bark product or extract is Enzogenol^{™}, Pycnogenol^{™}, or a combination thereof.

Typically the composition further includes chondroitinsulphate, or a chondroitin compound.

The composition preferably includes one or more anti-oxidants other than Enzogenol or equivalent bark extracts. More preferably said anti-oxidant is vitamin E.

The composition may also include deer velvet or a pharmacologically active extract thereof.

Preferably the composition includes additional glycosaminoglycans other than those present in the selected anti-inflammatory or enhancing agents.

Typically the composition comprises said green-lipped mussel extract (GLME) and/or a pharmacologically active green lipped mussel product in a sufficient amount to provide gastro-intestinal protection against irritation by other components in the composition.

The composition preferably includes one or more of the components selected from glycine, lysine, methionine, glutamic acid, tyrosine, and compounds providing in a pharmacologically acceptable form one or more of the following elements: manganese, zinc, iron, magnesium, selenium, calcium, copper, potassium, cobalt.

Preferably the composition is formulated to be suitable for addressing any one or more of the conditions in animals selected from inflammation, arthritis, chronic joint pain.

The composition may be used in any one or more of the following forms selected from a bolus or tablet, a capsule, a slow release implant, a liquid composition, a gel, or a paste.

The composition can also be formulated for use with non-human mammals.

According to a second aspect of the present invention there is provided the use of a composition as described in the first aspect for the preparation of a medicament for the treatment of joint problems in non-human animals.

Preferably said medicament is administered orally.

According to a third aspect of the present invention there is provided a combination of:
(i) a green-lipped mussel extract (GLME) and/or a pharmacologically active green lipped mussel product,
(ii) a shark cartilage and/or a pharmacologically active shark cartilage extract; and
(iii) an antioxidant bark extract,
for the preparation of a composition for use in addressing any one or more of:
(a) inflammation;
(b) degenerative joint complaints;
(c) other cartiligenous degeneration;
(d) gastrointestinal sensitivity or irritation;
(e) cancerous tumours.

The present invention has been developed with the needs of domesticated pets, and primarily mammalian species, in mind though it is also envisaged that the present invention is applicable to commercially bred species. However, while tablets or foodstuffs may be regularly administered or fed to pets or stabled animals, the problems associated with regular administration to sheep, cattle, and other livestock, may preclude regular use of the present invention with those species. However this does not mean that the present invention is detrimental, and therefore cannot be administered to such species or animals.

Preferred embodiments of the present invention focus around the use of three components, or equivalents thereof. These comprise green lipped mussel extract (GLME), shark cartilage and ENZOGENOL^{™}. Each of these components alone is known to exhibit a number of useful properties, though it has been found that varying combinations of these components can yield a significant improvement in the effectiveness of these components alone, and also render the resulting combination useful for addressing a number of complaints.

For instance, green lipped mussel extract (GLME) comprises extractions from the shellfish species *perna canaliculus,* a mollusc found on the shores of New Zealand. This is a convenient means for including active components from the green lipped mussel, though other forms of green lipped mussel and its products (preferably pharmacologically active) can be used. This mollusc has been found to contain a number of components exhibiting anti-inflammatory activity and includes small combinations of these components can yield a significant improvement in the effectiveness of these components alone, and also render the resulting combination useful for addressing a number of complaints.

For instance, green lipped mussel extract (GLME) comprises extractions from the shellfish species *perna canaliculus,* a mollusc found on the shores of New Zealand. This is a convenient means for including active components from the green lipped mussel, though other forms of green lipped mussel and its products (preferably pharmacologically active) can be used. This mollusc has been found to contain a number of components exhibiting anti-inflammatory activity and includes small amounts of glycosaminoglycans which have been shown to be beneficial for maintaining the integrity of cartilage and bone. Accordingly, green lipped mussel extract has been used for alleviating arthritic complaints, including degenerative joint diseases.

Green lipped mussel extract (GLME) where used in various embodiments of the present invention is preferentially that obtained from extraction processes from live, or recently killed mussels. Procedures such as outlined in granted patents to the inventor Stuart J McFarlane may be followed, though the product may preferentially be obtained from McFarlane Laboratories NZ Ltd., of New Zealand.

The same inventor has also pursued further patent applications directed to extracting specific targeted compounds from green lipped mussels, and re-combining or using these in other preparations. An example is the disclosure of US 4,455,298 (NZ 188489). Such extracts are also considered to be among the acceptable substitutes for green lipped mussel extract (GLME) for use in the present invention.

Shark cartilage has also been used by persons suffering from disorders such as cancer and arthritis and there it appears that it is useful in addressing these complaints. Identified active components include chondroitin sulphate, and glycosaminoglycans. Various shark cartilage products may be used, though preferentially include or retain active quantities of these components.

A further component which can be considered is a bark or plant extract exhibiting antioxidant properties. Preferably the antioxidant activity exceeds that of vitamin E. One product which has been mentioned is ENZOGENOL^{™}, a proprietary composition manufactured by Enzo Nutraceuticals Limited, of Christchurch, New Zealand, and comprises an extract from the bark of *Pinus radiata* which is rich in anti-oxidants. Other bark products exist with PYCNOGENOL^{™}, another proprietary product being an acceptable alternative. There is evidence establishing that oxidant and free radical damage can be addressed by this formulation. Both oxidant and free-radical damage have been shown to be involved in both premature ageing, and in particular, joint disease. Equivalent products to ENZOGENOL^{™} or PYCNOGENOL^{™} may be substituted, though the preference is for these products as they contain components other than antioxidants that may further enhance the properties of the product.

As previously indicated, it has been indicated that a significant useful improvement can be made by combining two or more of the three listed components. The selected combination will have some effect on the focus and activity of the resulting combination, and this will become more apparent from the following description.

One possible combination is green lipped mussel (GLM, and preferably an extract) with shark cartilage. This combination is of use as an anti-inflammatory, though in particular is useful for addressing arthritic complaints and degenerative joint problems. For instance, green lipped mussel and its preferred extracts include glycosaminoglycans which help protect cartilage and bone. Preferred GLM extracts also exhibit an anti-inflammatory effect. Most arthritic complaints and degenerative joint disorders are known to involve an associated inflammation in the joint region and thus extracts of GLM that have demonstrated effectiveness in these type of disorders have been at least partly attributable to the anti-inflammatory characteristics.

Shark cartilage contains higher levels of glycosaminoglycans which augment the cartilage protective effects of GLM products and extracts alone. This is further augmented by the presence of chondroitin sulphate, another cartilage protecting component. The collagen also present in shark cartilage further enhances the effectiveness of the combination.

Shark cartilage also possess some antiangiogenetic properties which also affords the combination and additional properties in addressing cancer tumour formation. It is also considered that the same property may also further enhance the ability of the combination to address, both preventatively, and curatively (to varying degrees) joint and cartilage problems - particularly mobility related ailments.

Enhancing agents such as ENZOGENOL^{™}, PYCNOGENOL^{™} or equivalent bark extracts, may also be combined with either or both of GLME and shark cartilage. Both GLME and_ shark cartilage possess anti-inflammatory properties. The combination with ENZOGENOL^{™}, with its anti-oxidant and anti-free radical properties, enhances the usefulness of these anti-inflammatories in addressing a number of disorders, and preventing the formation of other problems. For instance, inflammation is generally the consequence of a defensive action of the body and in some instances is accompanied by a significant amount of oxidants in the inflamed regions. These oxidants often include nitrous oxide, varying peroxides and a number of other substances which exhibit a strong localised anti-microbial effect. However, the effectiveness of their action is not always confined to foreign bodies. These oxidants produced by the body are also known to exhibit a negative effect on the host's own cells, and it is known that some oxidant species can disrupt host cell DNA sequences. Current theories consider this to be the first transformational change to occur in a number of forms of cancer, and thus addressing this problem will represent a preventative technique towards the establishment of a number of forms of cancer.

Anti-oxidants, such as those provided in ENZOGENOL^{™}, can reduce damage to the host's own cells, but without any significant decrease in the effectiveness of remaining oxidants in addressing microbial invaders and other-foreign-material. In some respects the anti-oxidants may be considered to have a regulating effect and tend to mop up excess oxidants which have been produced beyond the actual needs of the body.

Accordingly, the combination of a bark based anti-oxidant product with an anti-inflammatory, produces a substantially enhanced useful overall effect in reducing not only the amount of inflammation, but negative side effects associated with inflammation. Other factors may be at work though the use of products such as PYCNOGENOL^{™} or ENZOGENOL^{™} appear to confer the desired characteristics.

Further, the reduction in likelihood of an oxidant induced cancer transformation, coupled with the antiangiogenetic properties of shark cartilage, renders this a useful combination for reducing the probability of cancer formation.

Further, it will be appreciated that the combination of all three can yield a highly useful product which can help simultaneously address a number of afflictions which affect animals, and which become more prevalent in older animals.

Another anti-oxidant which may be used in varying embodiments of the present invention is vitamin E. Other anti-oxidants are also known, and both these and/or vitamin E may be used in varying embodiments including these combining GLM products and extracts with shark cartilage. However, preferred embodiments would include a bark based antioxidant as the preferred anti-oxidant of choice, though it should be also appreciated that not all uses of varying embodiments will focus on inflammation and its side-effects, and thus lower levels of additional anti-oxidant activity may be provided.

Other enhancing agents include plant based products exhibiting antioxidant properties, though may additionally, or alternatively, exhibit anti-inflammatory or anti-arthritic properties. In this later case, the preference is still to include an antioxidant, or to select a material also exhibiting antioxidant properties. One possibility is to include these other enhancing agents in combination with a bark based antioxidant such as ENZOGENOL^{™} or PYCNOGENOL^{™}. As a gauge of antioxidant activity, pharmacological activity comparable to or exceeding vitamin E is desirable, or alternatively an activity comparable to ENZOGENOL^{™} or PYCNOGENOL^{™}.

As can be appreciated, the varying combinations which have been described provide enhanced activity and properties over the individual components. The result is a range of embodiments which may be used in a number of similar roles, but which may exhibit slightly enhanced activity in one role over another.

Some of these components possess other useful properties which may extend the usefulness of various combinations. For instance, GLM products and extracts are known to be useful in preventing, alleviating, or treating gastro-intestinal irritation. Accordingly, compositions of the present invention which include GLM and/or its extracts may also be used as a carrier for, or as part of, compositions containing irritant substances just as GLME alone is used in such a role. This further extends the usefulness and flexibility of embodiments of the present invention.

For instance, many current fast-acting anti-inflammatories are irritating to the stomach. While embodiments of the present invention generally include sufficient anti-inflammatory activity, when administered over sustained periods, to preclude the use of most existing pharmaceutical anti-inflammatories, there may be instances where the user may wish or need to include one of these existing faster acting compounds. Including such a substance in such embodiments of the present invention may not only reduce the amount of the added anti-inflammatory which needs to be included, but the counter irritant effects of GLME can help reduce the side-effects from the administration of an added anti-inflammatory which may cause irritation.

There are a number of other pharmaceuticals which exhibit irritant properties, and the co-administration, or co-compounding, of embodiments of the present invention with those substances is also a technique within the scope of the present invention. In particular, embodiments of the present invention may find use for administration during chemotherapy which tends to have a number of significant negative side effects.

Embodiments of the present invention may also include other substances which are known to have a beneficial effect. One such substance is deer velvet for which a large amount of anecdotal, but little clinical, evidence exists of its effectiveness. The little clinical work which has been performed suggests that deer velvet administered orally can address problems associated with high blood pressure, as well as having both immuno-stimulatory and anti-inflammatory properties. The inclusion of deer velvet would therefore augment such properties already existing in various embodiments of the present invention.

It is also envisaged that varying embodiments-may-also include manganese ascorbate and/or S-adenosylmethionine (aka S-adenosyl-L-methionine 1,4 butane disulfonate). This latter compound is also known to promote joint mobility, while the former is involved in the biosynthesis of glycosaminoglycans. These can enhance the action of other components in preferred embodiments of the invention addressing debilitating joint ailments.

As mentioned previously, the present invention may take varying forms. It is envisaged that a common form of the invention is as an oral dosage form. This may be as a pill, tablet, capsule, etc. Liquid formulations may also be produced, as may other types of solid formulations. In particular, an animal foodstuff is envisaged. Each of these different forms may be prepared according to standard existing techniques, and which include the components of the various embodiments of the present invention.

### BEST MODES FOR CARRYING OUT THE INVENTION

### Example 1: Compositions for adult dogs

This comprises a tablet (or similar dosage form) or dietary foodstuff which includes green lipped mussel extract in combination with shark cartilage. Ideally, the composition also includes a range of vitamins and trace minerals in a balanced proportion, ideal for targeted animal range. Different embodiments may contain different ratios, depending upon the size, type, or age of the animal.

### Reference Example 1a: Constituents

In this embodiment, a dosage form, which may take the form of a pellet, capsule or tablet etc, may contain:

| | |
|---|---|
| Green Lipped Mussel Extract | 50 - 200 mg |
| Shark cartilage | 50 - 200 mg |
| Vitamin mix-optional but where included: | 200 ± 200 mg |

Which may, for example, consist of:

| | |
|---|---|
| Vitamin A | 2000-3000 iu |
| Vitamin D3 | 300-500 iu |
| Vitamin E | 20-30 iu |
| Vitamin K3 | 0.5-0.75 mg |
| Thiamine (Vitamin B1) | 1-1.5 mg |
| Riboflavin | 2-3 mg |
| Pyridoxine | 0.5-0.75 mg |
| Panthothenic acid | 2-3 mg |
| Niacin | 7-10.5 mg |
| Biotin | 0.1-0.75 mg |
| Vitamin B 12 | 22-150 µg |
| Folic acid | 0.1-0.15 mg |
| Iron | 12-20 mg |
| Copper | 1.5-2.5 mg |
| Cobalt | 0.25-0.4 mg |
| Manganese | 3-5 mg |
| Zinc | 25-40 mg |
| Iodine | 0.5-0.75 mg |
| Selenium | 0.075-0.125 mg |
| Calcium | 10-20 mg |
| Manganese ascorbate | optional |
| S-adenosylmethionine | optional |

The dosage form may also be incorporated into a food product, such as a pellet, which can be administered for consumption by the animal. Such dosage forms could also be seeded throughout pelletised animal foods - lower dosage forms may be prepared for such applications.

For the embodiment above, a typical suggested once daily dosage is:

| | |
|---|---|
| up to 15 kg | 1 tablets |
| 15 - 30 kg | 2 tablets |
| over 30 kg | 3 tablets |

This example is illustrative only. The vitamin mix is illustrative of a typical balance for adult dogs, but can be varied (and components added or eliminated) in different embodiments for other species and ages.

### Reference Example 1B

In this embodiment, a dosage form, which may take the form of a pellet, capsule or tablet etc, may contain:

| | |
|---|---|
| Green Lipped Mussel (preferably dried or powdered) or extract thereof: | 50 - 200 mg |
| Shark cartilage (preferably dried or powdered) or chondroitin sulphate or condroitin containing substance | 50 - 200 mg |
| Vitamin mix (as above in Example 1A) | optional |

The dosage form may also be incorporated into a food product, such as a pellet, which can be administered for consumption by the animal. Such dosage forms could also be seeded throughout pelletised animal foods - lower dosage forms may be prepared for such applications.

For the embodiment above, a typical suggested once daily dosage is:

| | |
|---|---|
| up to 15 kg | 1 tablets |
| 15 - 30 kg | 2 tablets |
| over 30 kg | 3 tablets |

This example is illustrative only. The vitamin mix is illustrative of a typical balance for adult dogs, but can be varied (and components added or eliminated) in different embodiments for other species and ages.

### Reference Example 2

This comprises a dosage form combining green lipped mussel with an anti-oxidant, and is of particular use for preventing or addressing inflammation.

In this embodiment a typical dosage form may contain:

| | |
|---|---|
| Green lipped mussel extract (or pharmacologically active green lipped mussel product) | 50 - 200 mg |
| ENZOGENOL^{™} or PYCNOGENOL^{™} | 5 ± 2 mg |
| Anti-inflammatory plant extract (optional) | 0 *-* 500 mg |
| Vitamin mix (see example 1 a) | 200 ± 200 mg. |

As for Example 1, the dosage form may take different forms, including capsules, tablets, pellets, and even liquid forms. Liquid forms would generally include an acceptable carrier, and may include inert oils such as comestible vegetable oils, and fish oils.

### Reference Example 3

This example combines shark cartilage with a bark based antioxidant. While this combination is useful for addressing inflammation, it is directed more to the prevention, and/or addressing arthritic complaints and degenerative joint diseases and afflictions.

In this embodiment the dosage form may contain:

| | |
|---|---|
| Shark cartilage | 50 - 200 mg |
| ENZOGENOL^{™} or PYCNOGENOL^{™} | 2 - 10 mg |
| Anti-arthritic and/or anti-inflammatory Plant extract (optional: | 0 - 500 mg |
| Vitamin mix (see example 1a) | 200 ± 200 mg. |

preferably including adenosylmethionine and manganese ascorbate.

Dosages and varying dosage forms, are as for the preceding examples.

### Reference Example 4

This embodiment includes deer velvet in addition to the compositions of any of the preceding examples. To a formulation as described in any of examples 1 through 3, there is also included deer velvet in the amount of 25 ±10 mg. Preferably this is dried deer velvet, which has been prepared by a method avoiding substantial degradation of included natural components.

Dosing and administration is as per Example 1 herein.

### Example 5: for older or arthritic animals, or animals exhibiting mobility problems

These embodiments may also be in dosage forms, or foodstuffs. This range of embodiments are targeted at older animals, and particularly those that may be showing joint problems or arthritis.

These embodiments combine green lipped mussel extract with shark cartilage or extracts thereof. Ideally, the shark cartilage, or any extract thereof, should include glycosaminoglycans. These two active components act as powerful anti-inflammatories, and provide anti-inflammatory action over the use of the green lipped mussel extract alone.

Optionally but ideally also, deer velvet or extract thereof is included in the these formulations.

Ideally also, these embodiments will also include ENZOGENOL (proprietary formulation of anti-oxidants).

### Example 5a: Constituents

Each tablet contains:

| | |
|---|---|
| Green Lipped Mussel Extract | 175 ± 75 mg |
| Deer Velvet | 25 ± 10 mg |
| Shark Cartilage | 100 ± 50 mg |
| ENZOGENOL (^{™}) or PYCNOGENOL^{™} | 5 ± 2 mg |
| Vitamin mix (see example 1a) | 200 ± 200 mg |

| | |
|---|---|
| Suggested once daily dosage as per example 1a. | |

May be fed in conjunction with Example 1a formulation. Can be administered directly into the mouth or added to the food.

### Example 6: for cats

The preferred embodiment for cats will include green lipped mussel extract. This acts in the role of an anti-inflammatory to improve mobility, as well as relief from sore and arthritic joints. Again, preferred embodiments of this range will also include a balanced range of vitamins and trace minerals for cats.

### Example 6a: Constituents

Each tablet contains:

| | |
|---|---|
| Green Lipped Mussel Extract (or pharmacologically active green lipped mussel product - quantity of such products may need to be varied according to activity) | 175 ± 75 mg |
| Either or both of: | |
| i) ENZOGENOL^{™} or PYCNOGENOL | 5 ± 2 mg |
| ii) shark cartilage | 20 - 175 mg |
| Taurine | 100 ± 50 mg |
| Potassium gluconate | 70 ± 20 mg |
| Thiamine hydrochloride | 25 ± 10 mg |
| Yeast | 50 ± 20 mg |
| Dextrose (as a tableting agent) | |
| Vitamin mix (see example 1a) | optional |

This composition can provide some additional benefit for cats. Taurine, an essential dietary ingredient in cats, is fundamental in preventing heart and eye disease. Taurine is also an important part of bile in the cat's digestive system. Potassium Gluconate helps prevent hypocalcaemia, a common diet related deficiency in cats.

Thiamine helps prevent diseases related to thiamine deficiency such as diarrhoea, kidney disease and polioencephomalcia. Yeast provides a rich source of B vitamins and other natural products. Dextrose is included as a tableting agent, instead of the more commonly used lactose, because many cats are lactose intolerant.

### Suggested once daily dosage

| | |
|---|---|
| 2.5 kg | 1 tablets |
| > 2.5 kg | 2 tablets |

Can be administered directly into the mouth or added to the food.

It is also possible to use the compositions of examples 1 through 5 for cats.

### Example 7

Trials were conducted using tablets on a number of different breeds of dog.

### Analysis of tablets used in trial

| Active ingredients: | per tablet |
|---|---|
| Green lipped mussel extract | 175mg |
| Shark cartilage | 100mg |
| ENZOGENOL^{™} | 5mg |

The natural ingredients contain traces of the following vitamins and minerals:

| | |
|---|---|
| Vitamin C | Tyrosine |
| Vitamin D3 | Potassium |
| Vitamin B1 | Cobalt |
| Vitamin B2 | Manganese |
| Niacin | Zinc |
| Vitamin B6 | Iron |
| Vitamin B12 | Magnesium |
| Glutamic acid | Selenium |
| Glycine | Calcium |
| Lysine | Copper |
| Methionine | |

The trial was an open assessment. The effect of the treatment was based on the owner's subjective observation of the dogs mobility and vitality. The patients chosen for treatment were dogs with lameness and/or diminished mobility due to pain from chronic arthritis. The recommended dose was 1 tablet per 10 kg bodyweight. The results of the trails are summarised in table 1. The effect is described as 0 : no effect, 1+ : some improvements, 2+ : good effect and 3+ : very good effect.

From table 1 it appears, that the recorded effect of the product in 12 out of 16 cases is good or very good. Typically there was seen improvement of mobility within 5 to 14 days, and especially it was noted by the clients that the dogs showed more vitality and improved well-being. This was most remarkable in geriatric patients.

The initial effect stabilises after 1 to 2 months. The owner also gets used to the better mobility of his dog. A certain depot-effect seems to be built up, which may last for weeks or months. Therefore it is recommended that there is a somewhat (50%) lower maintenance dose after initial dosing for approximately 2 months. After this period further improvement cannot be expected and the dog will stay in status quo.

It appears from table 1, that the indications mainly have been arthritis in different joints like elbow, knee, spondylosis etc. Clinically we have in a few cases observed diminished crepitation in arthritic joints, probably due to better lubrication. We have also observed better vitality in many cases.

During the trial were used tablets from 3 different batches. There was no noted difference as to quality or effect.

**Table 1: Race, indication, effect and number of glasses consumed in 16 dogs treated for joint pain**

| Race | Years | Indication | Dose/Day | Effect | Kg | Used glasses of 100 |
|---|---|---|---|---|---|---|
| Labrador | 11 | Arthroses+skin | 3 | 3+ | 34 | 5 |
| Labrador | 14 | Elbow arthrosis | 3 | 3+ | 26 | 5 |
| Labrador | 12 | Hip dysplasia | 2 | 3+ | 22 | 4 |
| Lab/ schæfer | 13 | Hip dysplasia/ knee | 2 | 3+ | 20 | 5 |
| Fox terrier | 14 | Shoulder arthrosis | 1 | 1+ | 8 | 1* |
| Dachshund | 14 | Spondylosis | 1 | 3+ | 8 | 3 |
| Shetl. sheepdog | 14 | Elbow arthrosis | 1 | 0 | 12 | 0** |
| Finsk Spids | 10 | Spondylosis | 2 | 3+ | 18 | 3 |
| Weimaraner | 6 | Cruc.rupt.chron. | 3 | 2+ | 26 | 1 |
| Border Collie | 0.5 | Cruc.rupt.acute | 1 | 2+ | 12 | 2 |
| Labrador | 0.4 | Hip dysplasia | 1 | 3+ | 18 | 3 |
| Golden Retriever | 8 | Knee arthros., skin | 3 | 2-3+ | 32 | 4 |
| Rottweiler | 4 | Elbow arthrosis | 3 | 2 | 38 | 4 |
| Schæfer | 14 | Spondylosis | 3 | 2+ | 32 | 3 |
| Labrador | 10 | Elbow arthrosis | 3 | 1+ | 28 | 6 |
| Boxer | 6 | Spondylosis | 3 | 1+ | 31 | 2 |

| | | | | | | |
|---|---|---|---|---|---|---|
| * Euthanised after 1 month due to Cushing syndrome. ** Medication stopped after 1 week due to polydipsia. | | | | | | |

Generally there were no observed adverse side-effects. One dog showed polyuri and polydipsia after 1 week treatment. The owner stopped treatment with the trial product and the symptoms disappeared. The dog was not examined as to the cause of the PU/PD, so the condition might have been due to other reasons.

### Conclusion

Chronic arthritis is very difficult to treat. The clinical response has been so positive, that this composition should be considered in future treatment of chronic arthrosis, of patients with loss of vitality and unspecified stiffness of joints or diminished mobility. For many dogs treatment with NASID or corticosteroids is problematic and in these cases many clients will prefer a natural, alternative treatment when a positive effect can be observed.

### Example 7

Following are the results of further efficacy tests performed using various embodiments of the present invention.

| No. | Dog Breed | Sex | Age (year) | Weight (kg) | Intake (tabs) | Symptom | Evaluation |
|---|---|---|---|---|---|---|---|
| 1. | Beagle | M(n) | 9 | 18.0 | 2 | Disk herniation | No effect |
| 2. | Akita | M | 11 | 31.8 | 3 | Knee arthritis | Remarkably effective |
| 3. | Miniature Dachshund | M(n) | 9 | 8.3 | 1 | Coxa aplasia | Effective |
| 4. | Mix | M | 11 | 9.8 | 1 | Patella luxation | Remarkably effective |
| 5. | Yorkshire Terrie | F | 13 | 2.2 | 1 | Coxa aplasia | Slightly effective |
| 6. | Sheltie | M | 13 | 11.8 | 1 | Coxa aplasia | Effective |
| 7. | Mix | F(h) | 11 | 17.4 | 2 | Knee arthritis | No effect |
| 8. | Sheltie | F(h) | 11 | 12.0 | 2 | Arthritis | Effective |
| 9. | Sheltie | F | 7 | 11.8 | 1 | Osteoarthritis of spine | Remarkably effective |
| 10. | Pomeranian | F(h) | | 4.7 | 1 | Coxa aplasia | No effect |
| 11. | Chow Chow | F | 3 | 38.2 | 3 | Carpus Arthritis | Remarkably effective |
| 12. | Mix | F | 3 | 29.7 | 2 | Traumatic Patella luxation | Remarkably effective |
| 13. | Pekinese | F | 14 | 5.6 | 1 | Coxa aplasia | No effect |
| 14. | Mix | F | 6 | 13.2 | 1 | Knee Arthritis | Effective |
| 15. | Sheltie | F | 8 | 17.0 | 2 | Arthritis | No effect |
| 16. | Pomeranian | F | 3 | 3.7 | 1 | Patella luxation | Effective |
| 17. | Maltese | F(h) | 10 | 4.7 | 1 | Arthritis | Remarkably effective |
| 18. | Bernese Mountain dog | F | 1 | 30.00 | 3 | Arthritis | Remarkably effective |
| 19. | Mix | M | 5 | 13.2 | 1 | Left hind foot lameness | Remarkably effective |

| No. | Dog Breed | Sex | Age (year) | Weight (kg) | Intake (tabs) | Symptom | Evaluation |
|---|---|---|---|---|---|---|---|
| 1. | Beagle | M(n) | 9 | 18.0 | 2 | Disk herniation | No effect |
| 2. | Akita | M | 11 | 31.8 | 3 | Knee arthritis | Remarkably effective |
| 3. | Miniature Dachshund | M(n) | 9 | 8.3 | 1 | Coxa aplasia | Effective |
| 4. | Mix | M | 11 | 9.8 | 1 | Patella luxation | Remarkably effective |
| 5. | Yorkshire Terrie | F | 13 | 2.2 | 1 | Coxa aplasia | Slightly effective |
| 6. | Sheltie | M | 13 | 11.8 | 1 | Coxa aplasia | Effective |
| 7. | Mix | F(h) | 11 | 17.4 | 2 | Knee arthritis | No effect |
| 8. | Sheltie | F(h) | 11 | 12.0 | 2 | Arthritis | Effective |
| 9. | Sheltie | F | 7 | 11.8 | 1 | Osteoarthritis of spine | Remarkably effective |
| 10. | Pomeranian | F(h) | | 4.7 | 1 | Coxa aplasia | No effect |
| 11. | Chow Chow | F | 3 | 38.2 | 3 | Carpus Arthritis | Remarkably effective |
| 12. | Mix | F | 3 | 29.7 | 2 | Traumatic Patella luxation | Remarkably effective |
| 13 | Pekinese | F | 14 | 5.6 | 1 | Coxa aplasia | No effect |
| 14. | Mix | F | 6 | 13.2 | 1 | Knee Arthritis | Effective |
| 15. | Sheltie | F | 8 | 17.0 | 2 | Arthritis | No effect |
| 16. | Pomeranian | F | 3 | 3.7 | 1 | Patella luxation | Effective |
| 17. | Maltese | F(h) | 10 | 4.7 | 1 | Arthritis | Remarkably effective |
| 18. | Bernese Mountain dog | F | 1 | 30.00 | 3 | Arthritis | Remarkably effective |
| 19. | Mix | M | 5 | 13.2 | 1 | Left hind foot lameness | Remarkably effective |

| No. | Dog Breed | Sex | Age (year) | Weight (kg) | Intake (tabs) | Symptom | Evaluation |
|---|---|---|---|---|---|---|---|
| 20. | Shiba | M | 12 | 10.5 | 2 | Osteoarthritis of spine | Exacerbation |
| 21. | Chihuahua | M(n) | 2 | | 1 | Arthritis | Remarkably effective |
| 22. | Mix | F(h) | 12 | 13.3 | 1 | Patella luxation | No effect |
| 23. | Golden retriever | F | 5 | 26.8 | 2 | Ligament rupture | Judgement impossible |
| 24. | Dachshund | F(h) | 8 | 4.3 | 1 | Arthritis | Slightly effective |
| 25. | Mix | F(h) | 12 | 8.7 | 1 | Osteoarthritis of spine | Remarkably effective |
| 26. | Mix | M(n) | 12 | 15.7 | 2 | Osteoarthritis of spine | Judgement impossible |
| 27. | Mix | F(h) | 9 | 19.9 | 2 | Coxalgia | Remarkably effective |
| 28. | Pug | F | 8 | 7.2 | 1 | Osteoarthritis of spine | No effect |
| 29. | Maltese | F | 18 | 3.0 | 1 | Left shoulder subluxation | Remarkably effective |
| 30. | Pomeranian | F(h) | 11 | 6.0 | 1 | Both hip arthritis deformans | Remarkably effective |
| 31. | Mix | F(h) | 9 | 13.2 | 1 | Both hip arthritis deformans | Remarkably effective |
| 32. | Shih Tzu | M(n) | 6 | 8.3 | 1 | Right patella luxation | Remarkably effective |

| No. | Dog Breed | Sex | Age (year) | Weight (kg) | Intake (tabs) | Symptom | Evaluation |
|---|---|---|---|---|---|---|---|
| 20. | Shiba | M | 12 | 10.5 | 2 | Osteoarthritis of spine | Exacerbation |
| 21. | Chihuahua | M(n) | 2 | | 1 | Arthritis | Remarkably effective |
| 22. | Mix | F(h) | 12 | 13.3 | 1 | Patella luxation | No effect |
| 23. | Golden retriever | F | 5 | 26.8 | 2 | Ligament rupture | Judgement impossible |
| 24. | Dachshund | F(h) | 8 | 4.3 | 1 | Arthritis | Slightly effective |
| 25. | Mix | F(h) | 12 | 8.7 | 1 | Osteoarthritis of spine | Remarkably effective |
| 26. | Mix | M(n) | 12 | 15.7 | 2 | Osteoarthritis of spine | Judgement impossible |
| 27. | Mix | F(h) | 9 | 19.9 | 2 | Coxalgia | Remarkably effective |
| 28. | Pug | F | 8 | 7.2 | 1 | Osteoarthritis of spine | No effect |
| 29. | Maltese | F | 18 | 3.0 | 1 | Left shoulder subluxation | Remarkably effective |
| 30. | Pomeranian | F(h) | 11 | 6.0 | 1 | Both hip arthritis deformans | Remarkably effective |
| 31. | Mix | F(h) | 9 | 13.2 | 1 | Both hip arthritis deformans | Remarkably effective |
| 32. | Shih Tzu | M(n) | 6 | 8.3 | 1 | Right patella luxation | Remarkably effective |
| 33. | Miniature Pinscher | M | 17 | 1.7 | 1 | Left elbow arthritis deformans | Judgement impossible |
| 34. | Pomeranian | M(n) | 7 | 6.3 | 1 | Left shoulder subluxation | Remarkably effective |
| 35. | Mix | F | 12 | 14.1 | 1 | Right hip and knee subluxation | Effective |
| 36. | Cavalier King Charles Spaniel | F | 3 | 7.9 | 1 | Left knee subluxation | Effective |

| | | |
|---|---|---|
| **Remarkably effective** | 48% | 16/33 |
| Every parameters were improvements, or more than 2 parameters improved 2 points | | |
| **Effective** | **21 %** | **7/33** |
| More than 2 parameters improved 1 point | | |
| **Minor response effective** | **6%** | **2/33** |
| More than 1 parameters improved 1 point | | |
| **Exacerbation** | **3%** | **1/33** |
| Taking a turn for the worse | | |
| No effect | 21% | 7/33 |
| No improvement | | |
| **Disable judgement** | 3 cases | |
| We could not evaluate (because of discontinuance) | | |

| | | | | | | | |
|---|---|---|---|---|---|---|---|
| 33. | Miniature Pinscher | M | 17 | 1.7 | 1 | Left arthritis deformans | Judgement impossible |
| 34. | Pomeranian | M(n) | 7 | 6.3 | 1 | Left shoulder subluxation | Remarkably effective |
| 35. | Mix | F | 12 | 14.1 | 1 | Right hip and knee subluxation | Effective |
| 36. | Cavalier King Spaniel | F | 3 | 7.9 | 1 | Left knee subluxation | Effective |

| | |
|---|---|
| Remarkably effective | 48% |
| Effective | 21% |
| Slightly effective | 6% |
| Exacerbation | 3% |
| No effect | 21 % |
| Judgement impossible | 3 cases |

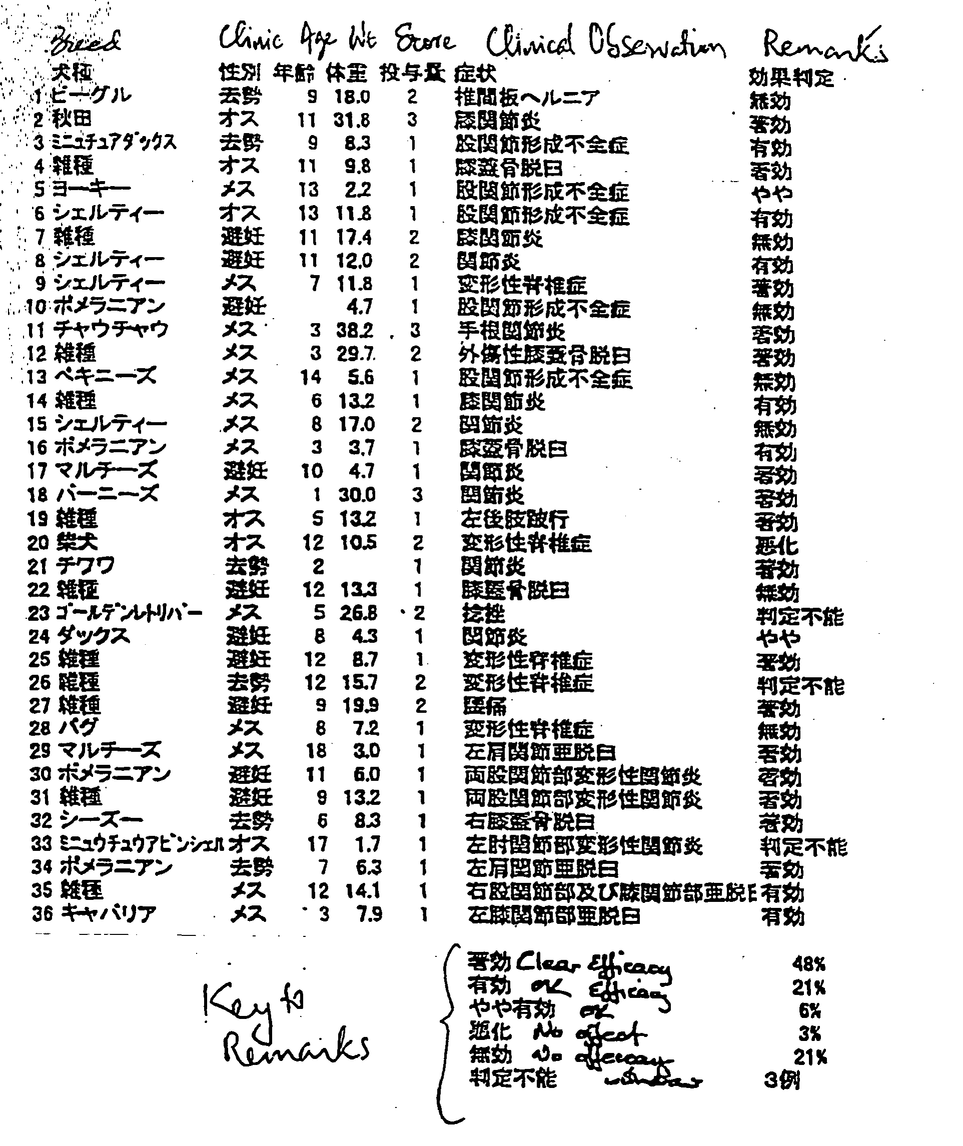

## Claims

1. An anti-inflammatory composition for administration to animals comprising a combination of:
a) a green-lipped mussel extract (GLME) and/or a pharmacologically active green lipped mussel product;
b) a shark cartilage and/or a pharmacologically active shark cartilage; and
c) a bark product or bark extract
wherein the bark product or extract enhances the effect of the anti-inflammatory agents.

2. A composition as claimed in Claim 1 which includes a range of vitamins and/or trace minerals.

3. A composition as claimed in either Claim 1 or Claim 2 wherein the bark product or extract is Enzogenol^{™}, Pyenogenol^{™}, or a combination thereof.

4. A composition as claimed in any of the preceding Claims further including chondroitin sulphate, or a chondroitin compound.

5. A composition as claimed in any one of the preceding Claims which includes one or more anti-oxidants other than Enzogenol or equivalent bark extracts.

6. A composition as claimed in Claim 5 in which said anti-oxidant is vitamin E.

7. A composition as claimed in any one of the preceding Claims which includes deer velvet or a pharmacologically active extract thereof.

8. A composition as claimed in any one of the preceding Claims which includes additional glycosaminoglycans other than those present in the selected anti-inflammatory or enhancing agents.

9. A composition as claimed in any one of the preceding Claims which comprises said green-lipped mussel extract (GLME) and/or a pharmacologically active green lipped mussel product in a sufficient amount to provide gastro-intestinal protection against irritation by other components in the composition.

10. A composition as claimed in any one of the preceding Claims which also includes one or more of the components selected from glycine, lysine, methionine, glutamic acid, tyrosine, and compounds providing in a pharmacologically acceptable form one or more of the following elements: manganese, zinc, iron, magnesium, selenium, calcium, copper, potassium, cobalt.

11. A composition as claimed in any one of the preceding Claims formulated to be suitable for addressing any one or more of the conditions in animals selected from inflammation, arthritis, chronic joint pain.

12. A composition as claimed in any one of the preceding Claims in any one or more of the following forms selected from a bolus or tablet, a capsule, a slow release implant, a liquid composition, a gel, or a paste.

13. A composition as claimed in any one of the preceding Claims formulated for use with non-human mammals.

14. The use of a composition as claimed in any of the preceding Claims for the preparation of a medicament for the treatment of joint problems in non-human animals.

15. The use as claimed in Claim 14 wherein said medicament is administered orally.

16. The use of a combination of:
(i) a green-lipped mussel extract (GLME) and/or a pharmacologically active green lipped mussel product,
(ii) a shark cartilage and/or a pharmacologically active shark cartilage extract;
and
(iii) an antioxidant bark extract,
for the preparation of a composition for use in addressing any one or mere of:
(a) inflammation;
(b) degenerative joint complaints;
(c) other cartiligenous degeneration;
(d) gastrointestinal sensitivity or irritation;
(e) cancerous tumours.

## Patentansprüche

1. Entzündungshemmendes Mittel zur Verabreichung an Tiere, umfassend eine Kombination aus
a) Grünlippmuschelextrakt (GLME) und/oder pharmakologisch aktivem Grünlippmuschelprodukt;
b) Haifischknorpel und/oder pharmakologisch aktivem Haifischknorpel;
c) Rindenprodukt oder Rindenextrakt,
wobei das Rindenprodukt oder der Rindenextrakt die Wirkung der entzündungshemmenden Wirkstoffe verbessert.

2. Mittel nach Anspruch 1, welches eine Reihe von Vitaminen und/oder Spurenelementen enthält.

3. Mittel nach Anspruch 1 oder Anspruch 2, wobei das Rindenprodukt oder der Rindenextrakt entweder Enzogenol^{™}, Pyenogenol^{™} oder eine Kombination davon ist.

4. Mittel nach einem der vorstehenden Ansprüche, weiterhin enthaltend Chondroitinsulfat oder eine Chondroitinverbindung.

5. Mittel nach einem der vorstehenden Ansprüche, welches ein oder mehrere Antioxidantien enthält, die weder Enzogenol noch gleichwertige Rindenextrakte sind.

6. Mittel nach Anspruch 5, bei dem das Antioxidanz Vitamin E ist.

7. Mittel nach einem der vorstehenden Ansprüche, welches Hirschbast oder einen pharmakologisch aktiven Extrakt davon enthält.

8. Mittel nach einem der vorstehenden Ansprüche, welches zusätzliche Glycosaminglycane enthält, die nicht bereits in den ausgewählten entzündungshemmenden oder die entzündungshemmende Wirkung verbessernden Wirkstoffen enthalten ist.

9. Mittel nach einem der vorstehenden Ansprüche, welches den Grünlippmuschelextrakt (GLME) und/oder ein pharmakologisch aktives Grünlippmuschelprodukt in ausreichender Menge umfasst, um den Magen-Darm-Trakt gegen Irritationen durch andere Bestandteile des Mittels zu schützen.

10. Mittel nach einem der vorstehenden Ansprüche, welches ebenfalls einen oder mehrere Bestandteile ausgewählt aus Glycin, Lysin, Methionin, Glutaminsäure, Tyrosin und Verbindungen enthält, welche in pharmakologisch akzeptabler Form eines oder mehrere der folgenden Elemente bereitstellt: Mangan, Zink, Eisen, Magnesium, Selen, Calcium, Kupfer, Kalium, Cobalt.

11. Mittel nach einem der vorstehenden Ansprüche, so formuliert, dass es zur Behandlung einer oder mehrerer Erkrankungen von Tieren wie Entzündungen, Arthritis und chronischen Gelenkschmerzen geeignet ist.

12. Mittel nach einem der vorstehenden Ansprüche, in einer oder mehreren der folgenden Formen, ausgewählt aus Bolus oder Tablette, Kapsel, Slow-release-Implantat, flüssigem Mittel, Gel oder Paste.

13. Mittel nach einem der vorstehenden Ansprüche, formuliert zur Verwendung bei nichtmenschlichen Säugetieren.

14. Verwendung eines Mittels nach einem der vorstehenden Ansprüche zur Zubereitung eines Medikaments zur Behandlung von Gelenkproblemen bei nichtmenschlichen Tieren.

15. Verwendung nach Anspruch 14, wobei das Medikament oral verabreicht wird.

16. Verwendung einer Kombination aus
(i) Grünlippmuschelextrakt (GLME) und/oder pharmakologisch aktivem Grünlippmuschelprodukt;
(ii) Haifischknorpel und/oder pharmakologisch aktivem Haifischknorpelextrakt; und
(iii) antioxidativ wirkendem Rindenextrakt,
zur Zubereitung eines Mittels zur Verwendung bei der Behandlung von:
(a) Entzündungen;
(b) degenerativen Gelenkbeschwerden;
(c) anderen Knorpeldegenerationen;
(d) Magen-Darm-Überempfindlichkeit oder -Irritationen;
(e) Krebstumoren.

## Revendications

1. Composition anti-inflammatoire destinée à être administrée à des animaux, comprenant un mélange de :
a) un extrait de moule verte (EMV) et/ou un produit de moule verte pharmacologiquement actif ;
b) du cartilage de requin et/ou du cartilage de requin pharmacologiquement actif ; et
c) un produit à base d'écorce ou un extrait d'écorce
dans laquelle le produit à base d'écorce ou extrait d'écorce accentue les effets des agents anti-inflammatoires.

2. Composition selon la revendication 1, comprenant une gamme de vitamines et/ou d'oligo-éléments.

3. Composition selon la revendication 1 ou 2, dans laquelle le produit à base d'écorce ou extrait d'écorce est de l'Enzogenol^{™}, du Pycnogenol^{™} ou un mélange des deux.

4. Composition selon l'une quelconque des revendications précédentes, comprenant en outre du sulfate de chondroïtine ou un composé de chondroïtine.

5. Composition selon l'une quelconque des revendications précédentes, comprenant un ou plusieurs antioxydants autres que l'Enzogenol ou extraits d'écorce équivalents.

6. Composition selon la revendication 5, dans laquelle ledit antioxydant est de la vitamine E.

7. Composition selon l'une quelconque des revendications précédentes, comprenant du velours de cerf ou un extrait pharmacologiquement actif de celui-ci.

8. Composition selon l'une quelconque des revendications précédentes, comprenant des glycosaminoglycanes supplémentaires autres que ceux présents dans les agents anti-inflammatoires ou d'accentuation sélectionnés.

9. Composition selon l'une quelconque des revendications précédentes, comprenant ledit extrait de moule verte (EMV) et/ou un produit de moule verte pharmacologiquement actif en quantité suffisante pour assurer une protection gastro-intestinale contre l'irritation par d'autres composants de la composition.

10. Composition selon l'une quelconque des revendications précédentes, comprenant également un ou plusieurs composants choisis parmi la glycine, la lysine, la méthionine, l'acide glutamique, la tyrosine, et des composés fournissant, sous une forme pharmacologiquement acceptable, un ou plusieurs des éléments suivants : manganèse, zinc, fer, magnésium, sélénium, calcium, cuivre, potassium, cobalt.

11. Composition selon l'une quelconque des revendications précédentes, formulée de manière à traiter l'une quelconque ou plusieurs des conditions, chez les animaux, sélectionnées parmi l'inflammation, l'arthrite et des douleurs articulaires chroniques.

12. Composition selon l'une quelconque des revendications précédentes, dans l'une quelconque ou plusieurs des formes suivantes choisies parmi un bolus ou un comprimé, une capsule, un implant à diffusion lente, une composition liquide, un gel ou une crème.

13. Composition selon l'une quelconque des revendications précédentes, formulée pour une utilisation chez des mammifères non humains.

14. Utilisation d'une composition selon l'une quelconque des revendications précédentes lors de la préparation d'un médicament destiné au traitement de problèmes articulaires chez des animaux non humains.

15. Utilisation selon la revendication 14, dans laquelle le médicament est administré par voie orale.

16. Utilisation d'un mélange de :
i) un extrait de moule verte (EMV) et/ou un produit de moule verte pharmacologiquement actif ;
ii) du cartilage de requin et/ou du cartilage de requin pharmacologiquement actif ; et
iii) un extrait d'écorce antioxydant,
lors de la préparation d'une composition destinée à être utilisée pour le traitement d'une ou plusieurs conditions parmi :
a) une inflammation ;
b) des problèmes articulaires dégénératifs ;
c) d'autres dégénérescences du cartilage ;
d) une sensibilité ou irritation gastro-intestinale ;
e) des tumeurs cancéreuses.
